# EUROPEAN PATENT APPLICATION

(11) **EP 4 495 605 A1**
(43) Date of publication of application: **22.01.2025**
(21) Application number: 23770360.8
(22) Date of filing: 27.02.2023
(51) Int. Cl.: G01N 35/10

(54) **TESTING DEVICE**

(30) Priority: 17.03.2022 JP 2022043032
(71) Applicant: FUJIFILM Corporation, Tokyo 106-8620 (JP)
(72) Inventor: MIURA, Yoshinobu, Ashigarakami-gun, Kanagawa 258-8538 (JP); TOKIWA, Nobuaki, Ashigarakami-gun, Kanagawa 258-8538 (JP)
(74) Representative: Dehns Germany Partnerschaft mbB
(86) International application number: PCT/JP2023/007053
(87) International publication number: WO 2023/176401

(57) **Abstract**

An examination apparatus includes: a detecting unit that detects a target substance in a specimen; a specimen dispensing unit that has a sampling nozzle, through which the specimen is suctioned from a specimen collection container and the specimen is discharged to a reaction cell and on which a chip is mounted at a distal end thereof, and a moving mechanism which moves the sampling nozzle; a nozzle imaging camera that captures an image of the sampling nozzle on which the chip is mounted; and a processor that is configured to control the moving mechanism and the nozzle imaging camera. The processor is configured to acquire the image of the sampling nozzle on which the chip is mounted from the nozzle imaging camera before the specimen is discharged to the reaction cell through the sampling nozzle, and the processor is configured to control the moving mechanism on the basis of a distal end position of the chip in the image such that the moving mechanism moves a distal end of the chip to a predetermined target discharge position inside the reaction cell.

## Description

### BACKGROUND OF THE INVENTION

### 1. Field of the Invention

The present disclosure relates to an examination apparatus.

### 2. Description of the Related Art

An examination apparatus that quantitatively or qualitatively detects a target substance in a specimen has been known. Many of such examination apparatuses use an immunoassay principle, and examples thereof include a chemiluminescent enzyme immunological analysis apparatus and a fluorescence immunological analysis apparatus (for example, JP2016-085093A).

Such an examination apparatus performs a detecting process of detecting a target substance in a specimen by detecting luminescence or fluorescence based on a label such as an enzyme label or a fluorescent label imparted to the target substance in the specimen by using an immunoreaction. Further, before such a detecting process of the target substance, a pre-process such as labeling the target substance in the specimen is performed on the specimen. In some cases, the examination apparatus is configured to automatically execute the pre-process and the detecting process and output the detection result in a case where the pre-process and the detecting process are automated and a specimen collection container accommodating the collected specimen is loaded.

In order to impart a label to the target substance in the specimen, for example, such an automated examination apparatus performs the following process using magnetic particles. First, in the reaction cell, the magnetic particles modified with the first binding substance (for example, the primary antibody) to specifically bind to the target substance (for example, the antigen) and the specimen are mixed with each other. Therefore, the target substance and the first binding substance are bound to each other to generate an immune complex. Thereby, the target substance is captured by the magnetic particles via the first binding substance. Thereafter, the immune complex is separated from the immune complex and a component derived from the specimen (unreacted substance) that does not form the immune complex, that is, so-called bound free (B/F) separation is performed. In a case of the B/F separation, the mixed liquid is suctioned in a state where the magnetic particles are temporarily adsorbed to an inner wall surface of the reaction cell by a magnet disposed outside the reaction cell. Thereafter, the cleaning liquid is discharged to the reaction cell, and the mixed liquid is suctioned and discharged in a state where the cleaning liquid and the magnetic particles are mixed. Therefore, the magnetic particles are cleaned. Next, a labeling reagent including a second binding substance (for example, a secondary antibody) to specifically bind to the target substance and the second binding substance bound to the label are mixed with the magnetic particles. Thereby, the target substance and the second binding substance captured on the magnetic particles via the first binding substance are bound to each other, whereby the sandwich type immune complex, in which the target substance is interposed between the first binding substance and the second binding substance, is generated. Thereafter, the magnetic particles are cleaned again by mixing the cleaning liquid and the magnetic particles for the B/F separation. In a case where the label is an enzyme label, the magnetic particles and the reagent including a luminescent substrate are further mixed and used for the examination process.

In such a manner, the automated examination apparatus is provided with a specimen dispensing unit that suctions a specimen from a specimen collection container and dispenses the specimen to the reaction cell and a reagent dispensing unit that dispenses various reagents to the reaction cell. In order to perform an accurate examination, it is necessary to dispense the specimen or the reagent to the reaction cell with certainty. Therefore, for example, JP2012-032309A proposes a configuration in which a position of a pipette (corresponding to a sampling nozzle) of a dispensing unit (corresponding to a pipetting unit) is adjusted on the basis of an image of the pipette captured by a camera.

On the other hand, JP2021-076392A discloses a system for preventing contamination of a distal end of a specimen dispensing rod (corresponding to a sampling nozzle) in a dispensing device applied to an examination apparatus or the like. The system disclosed in Patent Document 3 includes a configuration of comprising a state detecting unit, such as a camera, that detects a state where a filter portion and a chip portion are attached to a distal end of a specimen dispensing rod, and determines whether or not the filter portion and the chip portion are attached.

### SUMMARY OF THE INVENTION

In an examination apparatus that forms an immune complex using magnetic particles as described in JP2016-085093A to perform examination, in a case where the mixing of the magnetic particles and the specimen is not sufficient, an error may occur in the examination result.

In a case where the reaction cell accommodates a lump obtained by aggregating magnetic particles, by discharging the specimen to a lump of the magnetic particles, the lump is dissolved and the magnetic particles are easily dispersed. Thus, it is possible to suppress the failure in mixing between the magnetic particles and the specimen. However, in a case where a chip mounted on a distal end of a sampling nozzle is mounted in a tilted manner with respect to an axis of the sampling nozzle due to a mounting failure, for example, the magnetic particles may not be discharged toward the lump of the magnetic particles. In such a case, the lumps of the magnetic particles are not sufficiently dispersed. As a result, the magnetic particles and the specimen are not sufficiently mixed, which leads to an error in the examination result.

As described above, as disclosed in JP2012-032309A and JP2021-076392A, a configuration is known in which a camera that captures an image of a distal end of a sampling nozzle or a chip attached to the distal end is provided in a dispensing unit such as a pipetting unit provided in an automated examination apparatus. In addition, Patent Document 2 discloses adjusting a chip position with respect to the reaction cell. However, the accurate control of the discharge position in the reaction cell is not disclosed, and the viewpoint of suppressing the failure in mixing between the magnetic particles and the specimen is not described.

The present disclosure has been made in view of the above circumstances. An object of the present disclosure is to provide an examination apparatus capable of suppressing an error in an examination result and improving reliability of an examination by suppressing a failure in mixing between particles and a specimen.

An examination apparatus according to an aspect of the present disclosure comprises:
a detecting unit that detects a target substance in a specimen;
a specimen dispensing unit that has a sampling nozzle, through which the specimen is suctioned from a specimen collection container and the specimen is discharged to a reaction cell and on which a chip is mounted at a distal end thereof, and a moving mechanism which moves the sampling nozzle;
a nozzle imaging camera that captures an image of the sampling nozzle on which the chip is mounted; and
a processor that is configured to control the moving mechanism and the nozzle imaging camera,
in which the processor is configured to acquire the image of the sampling nozzle on which the chip is mounted from the nozzle imaging camera before the specimen is discharged to the reaction cell through the sampling nozzle, and
the processor is configured to control the moving mechanism on the basis of a distal end position of the chip in the image such that the moving mechanism moves a distal end of the chip to a predetermined target discharge position inside the reaction cell.

In the examination apparatus according to the aspect of the present disclosure, it is preferable that the target discharge position is a position which is at a center of the reaction cell in a horizontal direction and from which a bottom portion of the reaction cell is spaced by an interval predetermined as a reference in a vertical direction.

In the examination apparatus according to the aspect of the present disclosure, the processor may be configured to: acquire an image of the distal end of the chip by causing the nozzle imaging camera to capture the image before inserting the sampling nozzle into the reaction cell; derive, from the image, an amount of deviation between the distal end position of the chip and a predetermined appropriate position as a position at which the chip is appropriately mounted on the sampling nozzle; and control the moving mechanism such that the moving mechanism corrects the amount of deviation and moves the distal end of the chip to the target discharge position.

The examination apparatus according to the aspect of the present disclosure further may comprise a notification unit that issues a notification of an error, in which an allowable range may be set in which an amount of deviation between the distal end position of the chip and a predetermined appropriate position as a position at which the chip is appropriately mounted on the sampling nozzle is allowable, and the processor may be configured to: derive, from the image, the amount of deviation between the distal end position of the chip and the appropriate position; and stop movement of the sampling nozzle by the moving mechanism and cause the notification unit to issue the notification of the error, in a case where the amount of deviation is out of the allowable range.

The examination apparatus according to the aspect of the present disclosure may further comprise a reaction cell imaging camera that captures an image of the reaction cell, in which the reaction cell may accommodate a lump in which a plurality of magnetic particles are aggregated, and the processor may be configured to acquire the image of the reaction cell from the reaction cell imaging camera before the specimen is discharged to the reaction cell through the sampling nozzle, derive a position of the lump in the reaction cell from the image, and correct the target discharge position to a position facing the lump in a case where the lump deviates from a center of a bottom portion of the reaction cell.

In the examination apparatus according to the aspect of the present disclosure, the specimen dispensing unit may include a rotation mechanism that rotates the sampling nozzle about a length direction as an axis.

In the examination apparatus according to the aspect of the present disclosure, at least two cameras including a first camera and a second camera may be provided as the nozzle imaging camera, and the first camera and the second camera may be disposed around an axis at an interval of 90° in a case where a length direction of the sampling nozzle is set as the axis.

In the examination apparatus according to the aspect of the present disclosure, the processor may be configured to derive the distal end position of the chip in a depth direction from the nozzle imaging camera toward the chip and correct the target discharge position on the basis of a degree of optical blurring of the chip appearing in the image in addition to the distal end position of the chip in the image.

In the examination apparatus according to the aspect of the present disclosure, the processor may be configured to: acquire the image from the nozzle imaging camera before the specimen is suctioned through the sampling nozzle; and correct a suction position of the sampling nozzle in a case where the specimen is suctioned through the sampling nozzle by controlling the moving mechanism on the basis of the distal end position of the chip in the image.

In the examination apparatus according to the aspect of the present disclosure, the detecting unit may detect the target substance by detecting luminescence or fluorescence from a label attached to the target substance by using an antigen-antibody reaction.

According to the technology of the present disclosure, in the examination apparatus, it is possible to suppress the failure in mixing between the particles and the specimen, and to suppress the error of the examination result, thereby improving the reliability of the examination.

### BRIEF DESCRIPTION OF THE DRAWINGS

Fig. 1 is a schematic diagram showing a configuration of an examination apparatus 10 of a first embodiment.
Fig. 2A is a top view of a cartridge, and Fig. 2B is a front view thereof.
Fig. 3 is a diagram showing a step of imparting a label to a target substance in a reaction cell.
Fig. 4 is a diagram for describing a cleaning step.
Figs. 5A and 5B are diagrams showing a positional relationship between a nozzle imaging camera 61 and a sampling nozzle 42, where Fig. 5A is a plan view of a horizontal X-Y plane observed from a vertical direction, and Fig. 5B is a view of an X-Z plane perpendicular to a Y direction observed from the Y direction.
Fig. 6 is a diagram showing detailed steps of specimen dispensing.
Fig. 7 is a schematic diagram of a captured image PA which is acquired from the nozzle imaging camera 61.
Fig. 8 is a diagram showing a relationship between a distal end of a chip and a target discharge position in the reaction cell and a state of mixture between a liquid and magnetic particles in a case where the chip is appropriately mounted.
Fig. 9 is a diagram showing a relationship between the distal end of the chip and the target discharge position in the reaction cell and the state of mixture between the liquid and the magnetic particles in a case where the chip is mounted in a deviated manner.
Fig. 10 is a diagram showing a relationship between the distal end of the chip and the target discharge position in the reaction cell and the state of mixture between the liquid and the magnetic particles in a case where the chip is mounted in a deviated manner and the position correction is performed.
Figs. 11A and 11B are diagrams respectively showing a relationship between the chip and a specimen collection container at a suction position P1 in a case where the chip is mounted in a deviated manner, and a relationship between the chip and the specimen collection container after the correction of the suction position P1.
Fig. 12 is a schematic diagram showing a configuration of an examination apparatus 110 according to a second embodiment.
Fig. 13 is a diagram showing a positional relationship between a reaction cell imaging camera 62 and a reaction cell R0.
Fig. 14 is a diagram schematically showing images PB and PC of the reaction cell R0 of which an image is captured by the reaction cell imaging camera 62.
Fig. 15 is an explanatory diagram of a case where the chip is mounted in a deviated manner and the lump of magnetic particles in the reaction cell deviates from a center of a bottom portion.
Fig. 16 is an explanatory diagram of a rotation mechanism of a sampling nozzle.
Fig. 17 is a schematic diagram showing a configuration of an examination apparatus 120 of a third embodiment.
Fig. 18 is a plan view showing a positional relationship between a first camera 61A, a second camera 61B, and the sampling nozzle 42.
Fig. 19 is a diagram schematically showing an image PD of a chip 42A captured by the first camera 61A and an image PE of the chip 42A captured by the second camera 61B.
Fig. 20 is a diagram schematically showing an image PF and an image PG of the chip 42A captured by the nozzle imaging camera 61.

### DESCRIPTION OF THE PREFERRED EMBODIMENTS

Hereinafter, an examination apparatus according to an embodiment of the present disclosure will be described with reference to the drawings. In each of the drawings, constituent elements represented by the same reference numerals mean the same constituent elements. However, unless otherwise specified in the specification, each constituent element is not limited to one, and each constituent element may be plural.

### "Examination Apparatus according to First Embodiment"

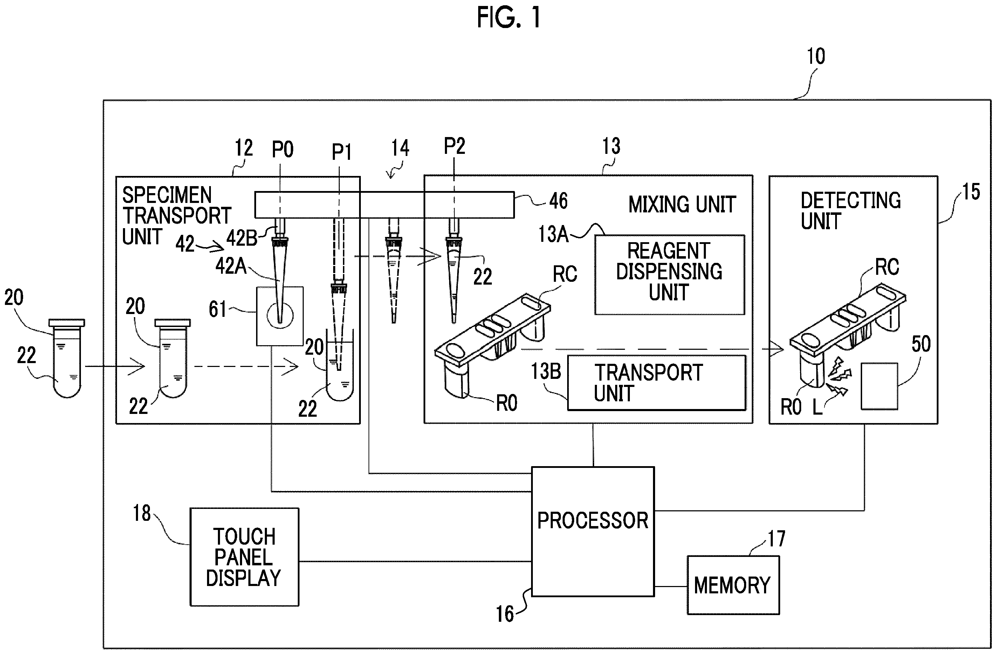

Fig. 1 is a schematic diagram showing a configuration of an immunological analysis apparatus which is an example of an examination apparatus 10 according to a first embodiment of the present disclosure. The examination apparatus 10 is an automatic immunological analysis apparatus that performs a pre-process of imparting a label to a target substance that is a substance to be detected in the specimen, performs a detecting process of detecting light from the label, and outputs a detection result, after a specimen collection container that accommodates a specimen collected from a biological body is loaded.

The examination apparatus 10 includes a specimen transport unit 12, a mixing unit 13, a specimen dispensing unit 14, a detecting unit 15, a processor 16, a memory 17, and a touch panel display 18.

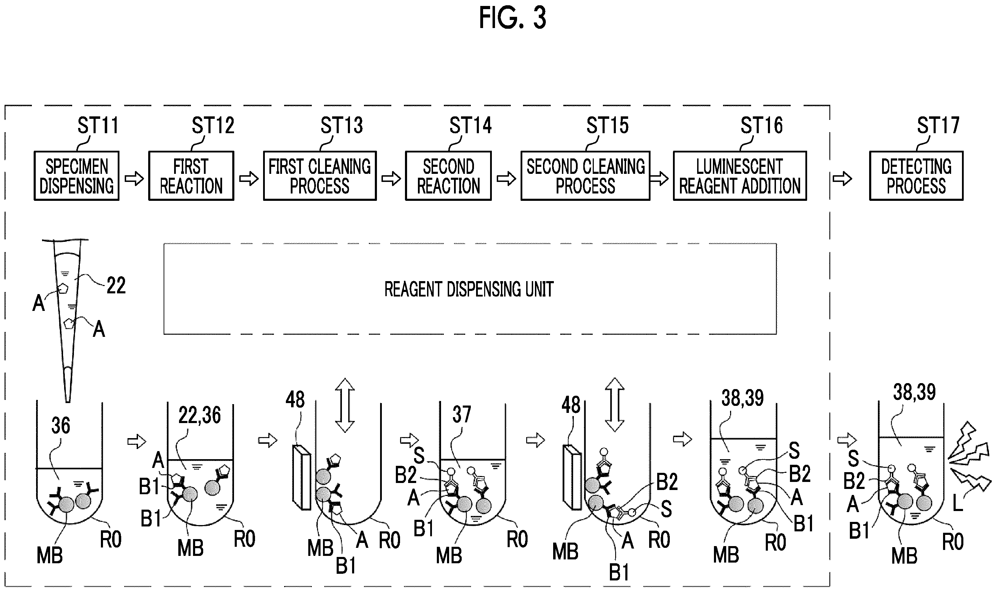

The detecting unit 15 executes a detecting process of detecting a target substance A (refer to Fig. 3 and the like) in a specimen 22. The detecting unit 15 includes a photodetector 50 such as a photomultiplier tube or a photodiode. The photodetector 50 is disposed to face a reaction cell R0 and detects light L caused by a label S bound to the target substance A. In the present example, an enzyme is used as the label S to detect chemiluminescence that is generated by reacting with the luminescent substrate.

A processor 16 integrally controls each unit of the examination apparatus 10. An example of the processor 16 is a central processing unit (CPU) that performs various types of control by executing a program. The CPU functions as a control unit that controls each unit by executing a program.

Further, the processor 16 acquires information about a light amount of the light L detected by the photodetector 50 and calculates a concentration of the target substance A on the basis of the information about the light amount.

A memory 17 is an example of a memory connected to or built into the CPU as the processor 16. For example, the memory 17 stores a control program. In addition to the control program, the memory 17 stores setting information that is preset in order for the processor 16 to perform the various types of control.

Further, the memory 17 stores information indicating a correspondence relationship between the light amount of the light L detected by the photodetector 50 and an amount of the target substance A. The correspondence relationship is stored, for example, as a calibration curve represented by a function. The correspondence relationship may be a format of a table. The processor 16 calculates the amount of the target substance A from, for example, the light amount of the light L, which is acquired from the photodetector 50, and the calibration curve stored in the memory 17.

The touch panel display 18 receives an operation instruction, such as an instruction to start an examination (hereinafter, referred to as an examination start instruction) by a user. Further, the touch panel display 18 displays information such as an examination result.

The specimen transport unit 12 has a loading section (not shown in the drawing) into which a specimen collection container 20 for accommodating the specimen 22 is loaded, and transports the loaded specimen collection container 20 to a position accessible by a sampling nozzle 42 of the specimen dispensing unit 14 to be described later.

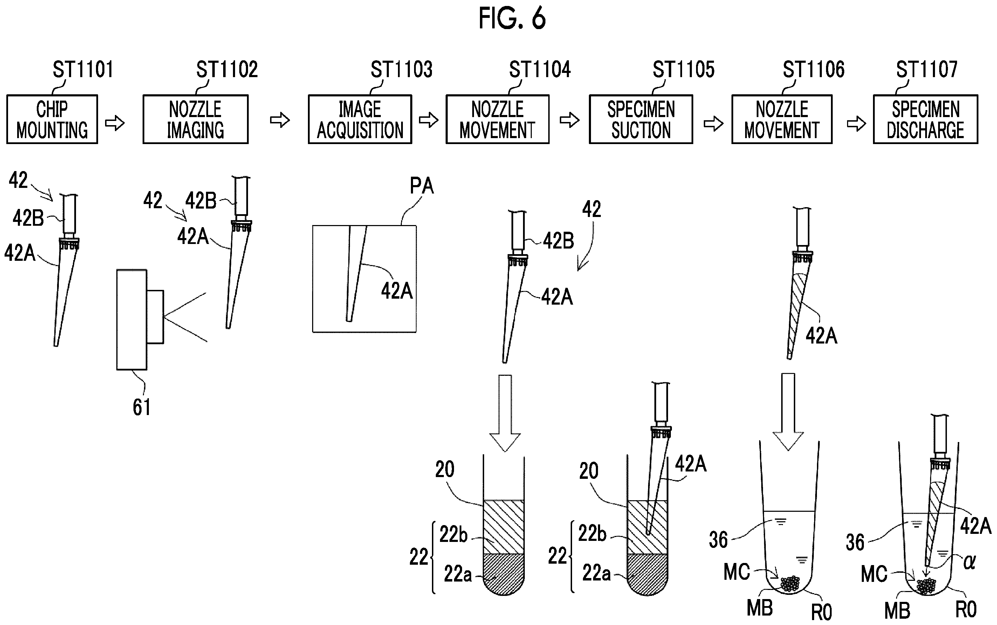

The specimen 22 is, for example, a biological fluid such as blood collected from a biological body. Hereinafter, a case where the specimen 22 is blood will be described, but the specimen 22 is not limited to blood. In the present embodiment, the specimen collection container 20 is a blood collection tube. For example, in a case where the blood is whole blood, the whole blood (specimen 22) is subjected to component separation into blood cells 22a and blood plasma 22b (refer to Fig. 6) or into blood clot and serum through the centrifugal separation process or the like, and the blood plasma or the serum is used for the examination of the target substance. The target substance, which can be included in the specimen 22 and which is a target of the examination, is, for example, an antigen, an antibody, a protein, or a low-molecular-weight compound.

The mixing unit 13 executes a mixing process of mixing the magnetic particles MB and the liquid in the reaction cell R0, the mixing process being performed on the specimen 22 to be used for the detecting process before the detecting process is performed. The mixing process will be described later. The mixing unit 13 includes a loading section (not shown in the drawing) into which a cartridge RC having a plurality of cells including the reaction cell R0 is loaded, and a reagent dispensing unit 13A. The cartridge RC is provided with cells R1 to R4 for accommodating various reagents in addition to the reaction cell R0, and openings 30 to 34 of the cells R1 to R4 are sealed with a sealing film (not shown in the drawing).

The reagent dispensing unit 13A includes a perforating nozzle (not shown in the drawing) having a perforating function of piercing the sealing film provided in the cartridge RC to open a hole. The mixing process is performed on the magnetic particles MB in the reaction cell R0 by repeating the suction and discharge of the liquid into which the magnetic particles MB are mixed by the perforating nozzle of the reagent dispensing unit 13A.

Further, the mixing unit 13 includes a transport unit 13B. The transport unit 13B transports the cartridge RC to a location at which each process step in the mixing unit 13 is executed. Further, the transport unit 13B transports the cartridge RC from the mixing unit 13 to the detecting unit 15.

The specimen dispensing unit 14 performs a specimen dispensing process of suctioning the specimen 22 from the specimen collection container 20 and dispensing the specimen 22 to the cartridge RC loaded in the mixing unit 13. The specimen dispensing unit 14 includes a sampling nozzle 42 and a moving mechanism 46 that moves the sampling nozzle 42. The moving mechanism 46 moves the sampling nozzle 42 in a vertical direction (up-down direction) and a horizontal direction. The movements of the sampling nozzle 42 in the vertical direction and the horizontal direction each are performed by a linear actuator as an example.

Further, for example, the sampling nozzle 42 includes a nozzle body 42B and a chip 42A that is interchangeably attached to a distal end of the nozzle body 42B. The distal end of the nozzle body 42B is configured to be aligned with a base end of the chip 42A. The chip 42A is replaced in order to prevent contamination from being caused by a plurality of liquids. The chip 42A is a single-use type and is disposable. The chip 42A of the sampling nozzle 42 is replaced for each specimen 22. In the present embodiment, the mounting of the chip 42A on the distal end of the nozzle body 42B of the sampling nozzle 42 is the same as the mounting of the chip 42A on the distal end of the sampling nozzle 42. The specimen dispensing unit 14 includes a loading section (not shown in the drawing) on which the chip 42A is loaded, and the loaded chip 42A is mounted on the nozzle body 42B of the sampling nozzle 42 at a mounting position P0.

The moving mechanism 46 moves the sampling nozzle 42 in the horizontal direction between the mounting position P0 of the chip 42A, a suction position P1 of the specimen transport unit 12, and a dispensing position P2 of the mixing unit 13. The mounting position P0 is a position at which the chip 42A is mounted on the distal end of the nozzle body 42B. The suction position P1 is a position at which the specimen 22 is suctioned from the specimen collection container 20. The dispensing position P2 is a position at which the specimen 22 is discharged to the reaction cell R0 of the cartridge RC which is set in the mixing unit 13.

Further, the moving mechanism 46 moves the sampling nozzle 42 in the vertical direction between an entrance position at which the chip 42A enters the specimen collection container 20 or the reaction cell R0 and a retreat position at which the chip 42A retreats from the specimen collection container 20 or the reaction cell R0 at each of the suction position P1 and the dispensing position P2. The liquid is suctioned and discharged through the sampling nozzles 42 at the entrance position.

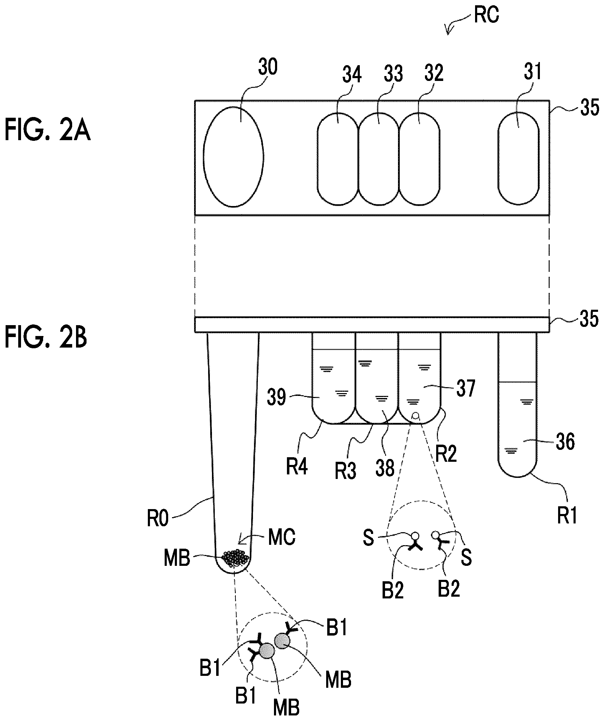

Fig. 2 is a schematic diagram of the cartridge RC, in which Fig. 2A is a top view of the cartridge RC and Fig. 2B is a front view of the cartridge RC. The cartridge RC includes a plate-shaped connection portion 35 having five openings 30 to 34, and five tubular cells R0 to R4 each having one end of each of the openings 30 to 34 and extending downward to include the reaction cell R0. The cartridge RC has a configuration in which a plurality of cells R0 to R4 are integrated by the connection portion 35. Among the plurality of cells R0 to R4, the reaction cell R0 and the cell R1 disposed at both ends are longer than the other cells R2 to R4. The reaction cell R0 is the longest.

Before use, the openings 30 to 34 of the cartridge RC are covered with the sealing film (not shown in the drawing). The cartridge RC is loaded in the examination apparatus 10 in advance, and one cartridge RC is used for one specimen 22.

The specimen 22 is dispensed to the reaction cell R0. The reaction cell R0 is a cell in which a process of imparting the label to the target substance in the specimen 22 is performed. The reaction cell R0 accommodates a lump MC of a plurality of magnetic particles MB modified with the first binding substance B1 to specifically bind to the target substance. In the reaction cell R0, the mixing process of mixing the magnetic particles MB and the liquid is performed. The mixing process of mixing the particles (here, the magnetic particles MB) and the liquid is a process of imparting the label to the target substance in the specimen 22. In a case where the magnetic particles MB each have a spherical shape, a diameter thereof is in a range of 0.1 to 10 µm, preferably 0.1 to 5 µm, and more preferably about 1 to 3 µm.

The cell R1 accommodates a buffer solution 36. The cell R2 accommodates a labeling reagent 37 including the label S in which a second binding substance B2 to specifically bind to the target substance is modified. A first luminescent reagent 38 is accommodated in the cell R3, and a second luminescent reagent 39 is accommodated in the cell R4. In the present example, the label S is an enzyme, and the label S emits light in presence of the first luminescent reagent 38 and the second luminescent reagent 39. The buffer solution 36 in the cell R1, the labeling reagent 37 in the cell R2, the first luminescent reagent 38 in the cell R3, and the second luminescent reagent 39 in the cell R4 are simply referred to as a reagent in a case where it is not necessary to distinguish the respective liquids.

The first binding substance B1 and the second binding substance B2, which specifically bind to the target substance, each are, for example, an antibody against an antigen in a case where the target substance is the antigen, an antigen against an antibody in a case where the target substance is the antibody, and an aptamer against a protein or a low-molecular-weight compound in a case where the target substance is the protein or the low-molecular-weight compound. The first binding substance B1 and the second binding substance B2 may be the same as or may be different from each other.

Here, a process of adding the label to the target substance in the specimen 22 in the reaction cell R0 will be described with reference to Fig. 3. A step surrounded by a broken line in Fig. 3 is a step performed in the mixing unit 13. Fig. 3 schematically shows reaction in the reaction cell R0. Here, a case where the specimen 22 includes the target substance A will be described.

First, the buffer solution 36 accommodated in the cell R1 is dispensed to the reaction cell R0 accommodating the magnetic particles MB modified with the first binding substance B1. Thereafter, the specimen 22 held by the sampling nozzle 42 is dispensed to the reaction cell R0 to which the buffer solution 36 has been dispensed (Step ST11).

In the reaction cell R0, the magnetic particles MB, the specimen 22, and the buffer solution 36 are mixed with one another. In the magnetic particles MB, the specimen 22, and the mixed liquid including the buffer solution 36, as the first reaction, a binding reaction, in which the target substance A in the specimen 22 and the first binding substance B1 specifically bind to each other, occurs (Step ST12). For example, in a case where the target substance A is an antigen, the first binding substance B1 is an antibody, and both the target substance A and the first binding substance B1 are bound to each other through an antigen-antibody reaction. In the first reaction, the target substance A in the specimen 22 binds to the first binding substance B1. Therefore, the target substance A is captured by the magnetic particles MB with the first binding substance B1 interposed therebetween.

Next, a first cleaning process (B/F separation) of removing an unreacted substance other than the target substance A, which is captured by the magnetic particles MB, is performed (Step ST13). In Step ST13 in Fig. 3, both arrows in the up-down direction shown at an upper portion of the reaction cell R0 schematically indicate a state where the discharge of the liquid in the reaction cell R0 and the supply of the liquid to the reaction cell R0 are performed.

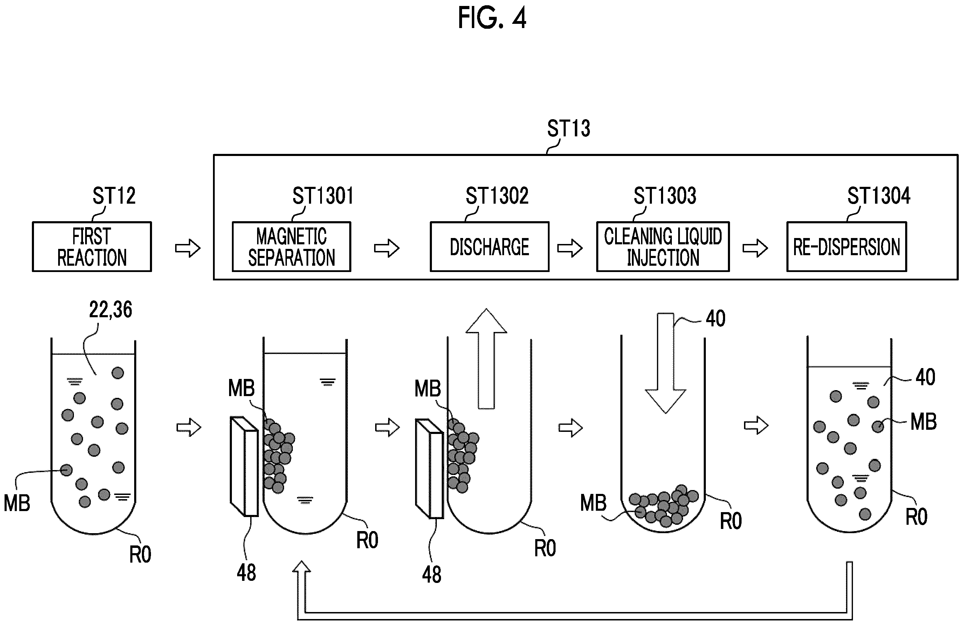

Details of the first cleaning process (Step ST13) will be described with reference to Fig. 4. Fig. 4 does not show the target substance A and the first binding substance B1.

The magnet 48 is disposed close to the outside of the reaction cell R0 in which the first reaction (Step ST12) is completed. Thereby, the magnetic particles MB in the reaction cell R0 are attracted to the magnet 48 and are collected on an inner wall surface of a side wall disposed close to the magnet 48, and magnetic separation of separating the magnetic particles MB and the liquid is performed (Step ST1301).

The liquid in the reaction cell R0 is discharged in a state where the magnetic particles MB are attracted to the inner wall surface of the reaction cell R0 (Step ST1302).

After the liquid is discharged from the reaction cell R0, the reaction cell R0 and the magnet 48 are separated from each other. In such a case, the reaction cell R0 and the magnet 48 are separated from each other such that magnetic force of the magnet 48 does not affect the magnetic particles MB in the reaction cell R0. Then, the cleaning liquid 40 is injected into the reaction cell R0 (Step ST1303).

After the cleaning liquid 40 is injected into the reaction cell R0, the mixed liquid in which the cleaning liquid 40 and the magnetic particles MB are mixed is suctioned and discharged, and the magnetic particles MB are re-dispersed in the cleaning liquid 40 (Step ST1304).

By repeating the steps from the magnetic separation (Step ST1301) to the redispersion (Step 1304) a plurality of times (for example, about 3 times), the unreacted substance other than the target substance A, which is captured by the magnetic particles MB, is removed. The B/F separation is performed by the above-mentioned cleaning step, and the magnetic particles MB and the target substance A, which is captured by the magnetic particles MB, remain in the reaction cell R0.

Returning to Fig. 3, the labeling reagent 37 accommodated in the cell R2 is dispensed to the reaction cell R0 subjected to the B/F separation as described above, and the magnetic particles MB and the labeling reagent 37 are mixed with each other.

In the mixed liquid containing the labeling reagent 37 and the magnetic particles MB, as the second reaction, a binding reaction, in which the target substance A captured by the magnetic particles MB and the second binding substance B2 specifically bind to each other, occurs (Step ST14). Thereby, the target substance A is interposed between the first binding substance B1 and the second binding substance B2, and the label S is applied to the target substance A with the second binding substance B2 interposed therebetween. For example, in a case where the target substance A is an antigen, the second binding substance B2 is an antibody, and both of the antigen and antibody are bound to each other through the antigen-antibody reaction. That is, in such a case, the label S is applied to the target substance A by using the antigen-antibody reaction.

Next, a second cleaning step (B/F separation) of removing the unreacted substance other than the second binding substance B2 which is bound to the target substance A and captured by the magnetic particles MB in the labeling reagent 37 is performed (Step ST15). In the second cleaning process (Step ST15), the two-way arrow in the up-down direction shown above the reaction cell R0 schematically indicates a state where the liquid in the reaction cell R0 is discharged and the liquid is supplied to the reaction cell R0, as in a case of Step ST13.

The second cleaning process (Step ST15) is performed in the same manner as the cleaning step performed in the first cleaning process (Step ST13). That is, the magnetic separation is performed by disposing the magnet 48 close to the outside of the reaction cell R0, the liquid in the reaction cell R0 is discharged, the cleaning liquid 40 is injected, and the magnetic particles MB are re-dispersed in the cleaning liquid 40 (refer to Fig. 4). Even in the second cleaning process (Step ST15), the step from magnetic separation to redispersion is repeated a plurality of times (for example, about 3 times). Thereby, the unreacted substance other than the label S applied to the target substance A, which is captured by the magnetic particles MB, is removed. The B/F separation is performed by the second cleaning process, and the magnetic particles MB, the target substance A, which is captured by the magnetic particles MB, and the label S, which is imparted to the target substance A, remain in the reaction cell R0.

The above-mentioned steps are steps of performing a process of imparting the label S to the target substance A in the specimen 22.

Thereafter, the first luminescent reagent 38 accommodated in the cell R3 and the second luminescent reagent 39 accommodated in the cell R4 are added to the reaction cell R0 (Step ST16). Thereby, the magnetic particles MB, the first luminescent reagent 38, and the second luminescent reagent 39 are mixed with one another.

The above-mentioned steps are a pre-process performed on the specimen 22 in the reaction cell R0 in the mixing unit 13.

The mixing unit 13 performs the above-mentioned pre-process, and the reagent dispensing unit 13A suctions and discharges various liquids. For example, as described above, the cleaning liquid 40 is suctioned from the cleaning liquid accommodating portion that accommodates the cleaning liquid 40, and the suctioned cleaning liquid 40 is discharged to the reaction cell R0, or each of the reagents is suctioned from each of the cells R1 to R4, and the suctioned reagent is discharged to the reaction cell R0.

The cartridge RC, on which the above-mentioned pre-process is completed, is transported to the detecting unit 15 by the transport unit 13B and is subjected to the detecting process in the detecting unit 15. In the reaction cell R0, the label S, which is imparted to the target substance A, reacts with the first luminescent reagent 38 and the second luminescent reagent 39, which are the luminescent substrates added in Step ST16, to generate chemiluminescence L. The photodetector 50 detects the chemiluminescence L.

As shown in Fig. 1, the examination apparatus 10 includes a nozzle imaging camera 61 that captures an image of the sampling nozzle 42 on which the chip 42A is mounted. The processor 16 controls the nozzle imaging camera 61. The processor 16 acquires the image of the sampling nozzle 42 on which the chip 42A is mounted from the nozzle imaging camera 61 before the discharging of the specimen 22 to the reaction cell R0 of the specimen 22 through the sampling nozzle 42. Then, the processor 16 controls the moving mechanism 46 such that the moving mechanism 46 moves the distal end of the chip 42A to a predetermined target discharge position α inside the reaction cell R0 on the basis of the distal end position of the chip 42A in the image.

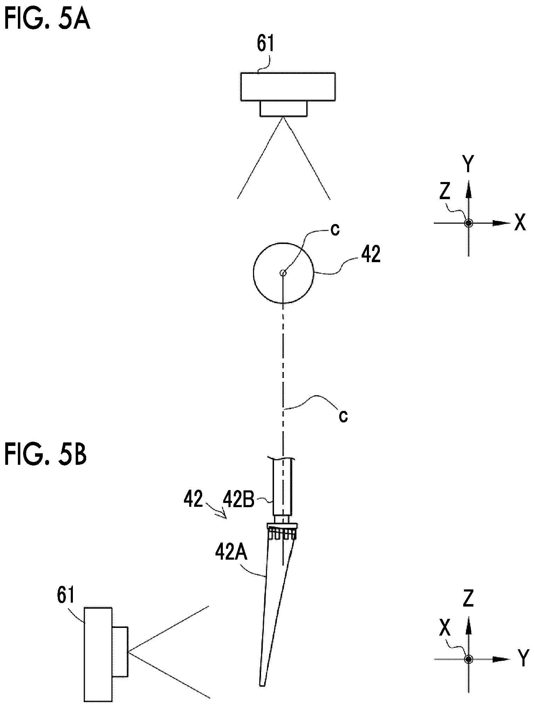

Fig. 5 is a diagram showing a positional relationship between the nozzle imaging camera 61 and the sampling nozzle 42. Specifically, Fig. 5A is a plan view in a case where a horizontal X-Y plane is observed from the vertical direction, and Fig. 5B is a view in a case where an X-Z plane perpendicular to a Y direction is observed from the Y direction. Here, a horizontal movement direction of the sampling nozzle 42 moved by the moving mechanism 46 is defined as an X direction, a direction, which is a horizontal direction and is orthogonal to the X direction, is defined as the Y direction, and a vertical direction (up-down direction), which is orthogonal to the XY plane, is defined as a Z direction. The directions indicated by the arrows X, Y, and Z in respective figures coincide with each other.

As shown in Figs. 5A and 5B, the nozzle imaging camera 61 is disposed at a position at which an image of the chip 42A mounted on the distal end of the nozzle body 42B of the sampling nozzle 42 can be captured. In the present embodiment, as shown in Figs. 5A and 5B, the nozzle imaging camera 61 is disposed such that the image of the chip 42A can be captured at the mounting position P0 (refer to Fig. 1) at which the chip 42A is mounted on the nozzle body 42B, thereby capturing the image of the chip 42A on the X-Z plane perpendicular to the Y direction.

In the examination apparatus 10, the specimen dispensing Step ST11 from the mounting of the chip 42A on the sampling nozzle 42 to the discharging of the specimen 22 into the reaction cell R0 will be described with reference to Fig. 6.

First, at the chip mounting position P0 (refer to Fig. 1) in the examination apparatus 10, the chip 42A is mounted on the distal end of the sampling nozzle 42 which is, here, the distal end of the nozzle body 42B (Step ST1101).

Next, the processor 16 controls the nozzle imaging camera 61 such that the nozzle imaging camera 61 captures the image of the sampling nozzle 42 at the mounting position P0 (Step ST1102). Here, for example, the nozzle imaging camera 61 captures the image of the distal end of the chip 42A of the sampling nozzle 42.

The processor 16 acquires an image PA including the distal end of the chip 42A from the nozzle imaging camera 61 and detects a nozzle distal end position from the image PA (Step ST1103).

The processor 16 controls the moving mechanism 46 such that the moving mechanism 46 horizontally moves the sampling nozzle 42 from the mounting position P0 to the suction position P1, and then moves the sampling nozzle 42 vertically downward (Step ST1104) and inserts the chip 42A into the specimen collection container 20. Thereby, the distal end of the chip 42A is positioned at a predetermined suction position, and the specimen 22 is suctioned (Step ST1105).

The processor 16 controls the moving mechanism 46 such that the moving mechanism 46 moves the sampling nozzle 42 vertically upward after suctioning the specimen 22 through the sampling nozzle 42, horizontally moves the sampling nozzle 42 from the suction position P1 to the dispensing position P2, and further moves the sampling nozzle 42 vertically downward (Step ST1106). In such a case, the processor 16 controls the moving mechanism 46 such that the moving mechanism 46 moves the distal end of the chip 42A to the predetermined target discharge position α inside the reaction cell R0 on the basis of the distal end position of the chip 42A in the image PA acquired from the nozzle imaging camera 61.

The processor 16 causes the specimen 22 to be discharged from the chip 42A in a state where the distal end position of the chip 42A is positioned at the target discharge position α (Step ST1107).

A specific embodiment, in which the correction is performed on the basis of the distal end position of the chip 42A in the image PA, will be described. In the present embodiment, the nozzle imaging camera 61 is disposed to be able to capture an image of the distal end of the chip 42A at the mounting position P0 of the chip 42A, and is set to perform focusing on the chip 42A in a case where the chip 42A is appropriately mounted on the sampling nozzle 42 at the mounting position P0. The processor 16 controls the nozzle imaging camera 61 such that the nozzle imaging camera 61 captures the image of the distal end of the chip 42A at the mounting position P0. Therefore, in the present embodiment, the imaging by the nozzle imaging camera 61 is performed before the sampling nozzle 42 is moved to the suction position P1. However, the configuration of the nozzle imaging camera 61 is not limited to the configuration in which the image of the distal end of the chip 42A is captured at the mounting position P0. The nozzle imaging camera 61 may be disposed such that the image in the state where the chip 42A is mounted can be captured before the sampling nozzle 42 is inserted into the reaction cell R0. That is, the imaging of the distal end of the chip 42A by the nozzle imaging camera 61 may be performed before the sampling nozzle 42 is inserted into the reaction cell R0.

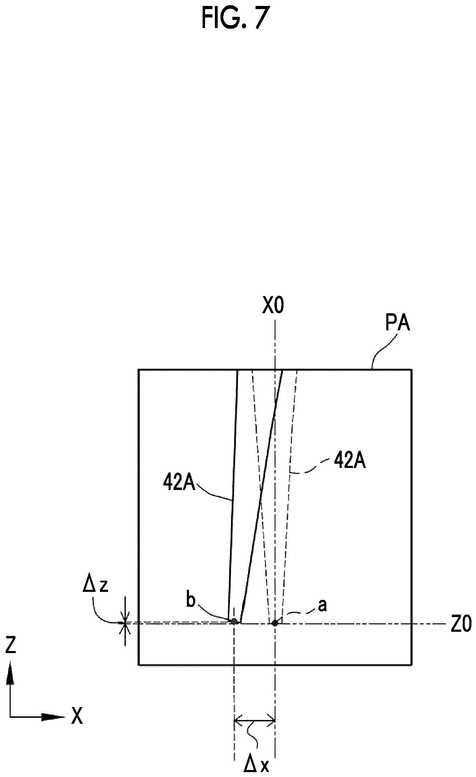

The processor 16 derives an amount of deviation between the distal end position of the chip 42A and a predetermined appropriate position as a position at which the chip 42A is appropriately mounted on the sampling nozzle 42 from the image PA acquired from the nozzle imaging camera 61. Fig. 7 is a diagram schematically showing the image PA of the distal end of the chip 42A captured by the nozzle imaging camera 61.

In Fig. 7, a position indicated by a broken line is a position at which the image of the chip 42A is captured in a case where the image of the distal end of the chip 42A is captured by the nozzle imaging camera 61 in a state where the chip 42A is appropriately mounted on the sampling nozzle 42 (here, the nozzle body 42B). The distal end position of the chip 42A that is appropriately mounted is stored in the memory 17 as a predetermined appropriate position a. In the present example, the appropriate position a is at the coordinates (X0, Z0) in the image PA. The processor 16 derives an amount of deviation (Δx, Δz) between the distal end position b of the chip 42A at the time of imaging and the appropriate position a, which is indicated by a solid line in Fig. 7. In a case where the actual chip 42A is appropriately mounted on the nozzle body 42B, a distal end position b and the appropriate position a match. As shown in Fig. 5, in a case where the chip 42A is disposed to be inclined with respect to an axis c of the nozzle body 42B, the distal end position b of the chip 42A deviates from the appropriate position a as shown in Fig. 7.

The processor 16 controls the moving mechanism 46 such that the moving mechanism 46 corrects an amount of movement, by which the sampling nozzle 42 is moved, in accordance with the amount of deviation to move the distal end of the chip 42A to the target discharge position α.

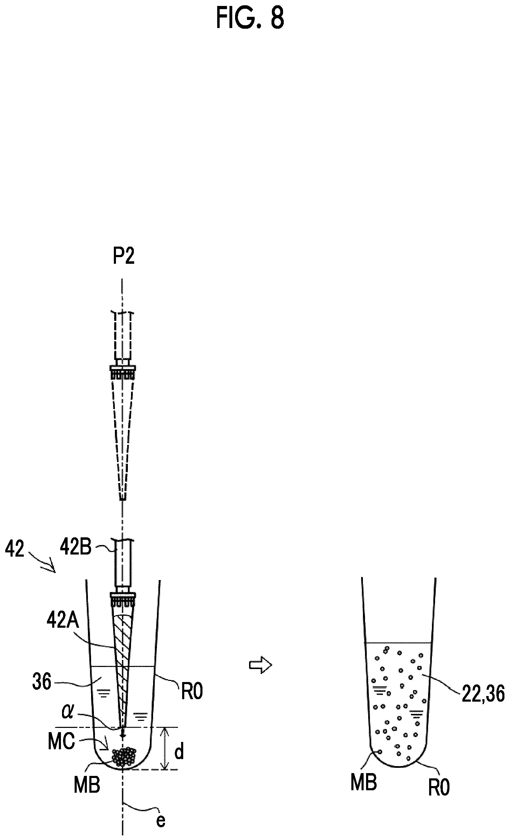

In a case where the chip 42A is appropriately mounted on the sampling nozzle 42, the processor 16 controls the moving mechanism 46 such that the moving mechanism 46 positions the distal end of the chip 42A at a predetermined target discharge position α inside the reaction cell R0 as shown in Fig. 8. The memory 17 stores, as an amount of horizontal movement from the suction position P1 to the dispensing position P2 and an amount of vertical movement in the vertical direction at the dispensing position P2, a prescribed amount of movement in a case where the chip 42A is appropriately mounted on the sampling nozzle 42. In a case where the chip 42A is appropriately mounted on the sampling nozzle 42, the dispensing position P2 substantially coincides with a central axis e of the reaction cell R0. The processor 16 controls the moving mechanism 46 on the basis of the prescribed amount of movement such that the moving mechanism 46 moves the sampling nozzle 42 to position the distal end of the chip 42A at the target discharge position α.

The target discharge position α is a position in the vicinity of the center of the bottom portion of the reaction cell R0, on the central axis e of the reaction cell R0 in the horizontal direction, and at a position from which the bottom portion of the reaction cell R0 is spaced by a interval d predetermined as a reference in the vertical direction. Here, the vicinity of the center of the bottom portion is defined as a range of a radius of 2 mm centered on the central axis e, with the interval d of 3 mm or less. In a case where the distal end of the chip 42A is positioned at the target discharge position α, the sampling nozzle 42 discharges the specimen 22 toward the lump MC of the magnetic particles MB positioned at the center of the bottom portion of the reaction cell R0. Thereby, the lump MC of the magnetic particles MB is dissolved by receiving a water pressure caused by the discharging of the specimen 22, and the individual magnetic particles MB are easily dispersed in the liquid. The magnetic particles MB are uniformly dispersed in the specimen 22 and the buffer solution 36 by repeating the discharging and the suctioning of the chip 42A at the target discharge position α a plurality of times.

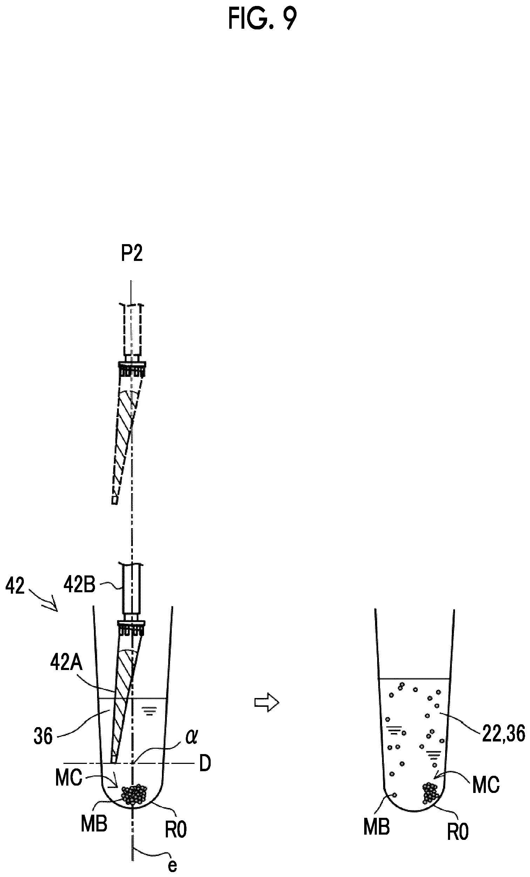

On the other hand, as shown in Fig. 9, in a case where the chip 42A is mounted on the sampling nozzle 42 in an inclined manner, and the sampling nozzle 42 is moved by a prescribed amount of movement, the distal end position of the chip 42A is a position deviated from the target discharge position α. In such a case, the specimen 22, which is discharged from the distal end of the chip 42A of the sampling nozzle 42, is discharged toward a wall surface of the reaction cell R0 without being discharged toward the lump MC. Therefore, in some cases, the water pressure at the time of discharging the specimen 22 may not be sufficiently applied to the lump MC, and it may be difficult to sufficiently dissolve the lump MC. Therefore, even in a case where the discharging and the suction are repeated a plurality of times through the sampling nozzle 42, in some cases, the lumps MC may not be sufficiently separated in the specimen 22 and the buffer solution 36, the lumps MC may remain, and the dispersion of the magnetic particles MB may be insufficient. The magnetic particles MB are not sufficiently mixed with the specimen 22 and the buffer solution 36.

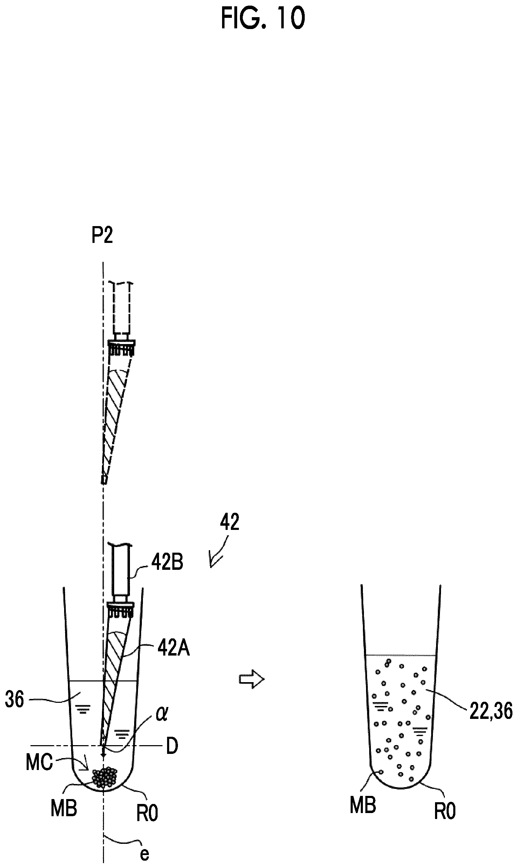

In the present embodiment, as shown in Fig. 10, in a case where the chip 42A is mounted on the sampling nozzle 42 in an inclined manner, the processor 16 corrects the prescribed amount of movement and controls the moving mechanism 46 such that the moving mechanism 46 positions the distal end of the chip 42A at the target discharge position α.

As described above, in the examination apparatus 10, the processor 16 acquires the image of the state where the chip 42A is mounted on the distal end of the sampling nozzle 42 from the nozzle imaging camera 61 before the specimen 22 is discharged to the reaction cell R0 through the sampling nozzle 42. Then, the sampling nozzle 42 is moved by the moving mechanism 46 on the basis of the distal end position of the chip 42A derived from the acquired image. Thereby the distal end of the chip 42A is moved to a predetermined target discharge position α inside the reaction cell R0. Thereby, the specimen 22 can be discharged at the target discharge position α of the reaction cell R0 regardless of the mounting state of the chip 42A. In a case where a lump MC of magnetic particles MB aggregated at the center of the bottom portion of the reaction cell R0 is present, the specimen 22 can be discharged toward the lump MC, and the lump MC can be dissolved. Thus, it is possible to easily disperse the magnetic particles MB. It is possible to suppress the failure in mixing and improve the reliability of the examination.

Further, as in the present embodiment, in a case where the target discharge position α is a position at which the reaction cell R0 is provided at the center in the horizontal direction and is provided at a position from which the bottom portion of the reaction cell R0 is spaced by the interval d predetermined as a reference in the vertical direction, the specimen 22 can be discharged toward the center of the bottom portion of the reaction cell R0. In a case where the lump MC of the magnetic particles MB is disposed at the center of the bottom portion in the reaction cell R0, the specimen 22 can be discharged toward the lump MC. Therefore, the lump MC is dissolved by the water pressure which is applied to the lump MC by the specimen 22 discharged from the discharging port. Thus, it is possible to easily disperse the individual magnetic particles MB.

Further, in the examination apparatus 10 of the present embodiment, the processor 16 acquires an image PA of the sampling nozzle 42, which is an image including at least the distal end of the chip 42A, by causing the nozzle imaging camera 61 such that the nozzle imaging camera 61 captures an image of the distal end of the chip 42A before the sampling nozzle 42 is inserted into the reaction cell R0. Then, the processor 16 derives the amount of deviation between the distal end position b of the chip 42A and the appropriate position a, corrects the amount of deviation by the moving mechanism 46, and moves the sampling nozzle 42 such that the distal end of the chip 42A is positioned at the target discharge position α. However, the processor 16 may be configured to derive the distal end position b of the chip 42A, derive the amount of movement to the position of the target discharge position α, and cause the moving mechanism 46 to move the sampling nozzle 42. In the present embodiment, the prescribed amount of movement in a case of moving the chip 42A to the target discharge position is corrected in accordance with the amount of deviation between the distal end position b of the chip 42A and the appropriate position a. Therefore, it is possible to reduce the processing load of the processor 16.

It should be noted that an allowable range, in which an amount of deviation between the distal end position b of the chip 42A is allowable, and the appropriate position a may be set. The memory 17 may store the allowable range. In a case where the amount of deviation is within the allowable range, as described above, the processor 16 corrects the deviation and performs the movement. In contrast, in a case where the amount of deviation is out of the allowable range, the processor 16 may be configured to cancel the movement of the sampling nozzle 42 performed by the moving mechanism 46 and display an error message indicating that the chip 42A deviates by more than the allowable range on the touch panel display 18. In such a case, the touch panel display 18 functions as a notification unit that issues a notification that the mounting state of the chip 42A is unfavorable. As a notification unit that issues a notification that the mounting state of the chip 42A is unfavorable, a speaker that issues a notification of an error by voice or a light emitting unit that issues a notification of an error by emitting light may be provided separately from the touch panel display 18.

The allowable range, in which the amount of deviation between the distal end position b and the appropriate position a of the chip 42A is allowable, includes, for example, an amount of deviation by which the distal end of the chip 42A can be moved to the target discharge position α through the correction. The allowable range is set such that the amount of deviation is out of the allowable range in a case where the inclination of the chip 42A with respect to the axis c of the nozzle body 42B is excessively large and the chip 42A collides with the wall surface of the reaction cell R0 and is unlikely to be inserted into the reaction cell R0 due to correction of the amount of movement.

In such a manner, in a case where it is difficult to position the chip 42A at the target discharge position α in the correction of the amount of movement performed by the moving mechanism 46, it is preferable that the configuration is made to issue a notification of an error. By promptly reporting an error in a case where a deviation which is uncorrectable occurs, it is possible to prevent an examination from being performed while a failure in mixing occurs, and it is possible to improve reliability of the examination.

In the examination apparatus 10 of the present embodiment, the processor 16 moves the distal end of the chip 42A to the predetermined target discharge position α inside the reaction cell R0, on the basis of the distal end position of the chip 42A in the image PA obtained by capturing the image of the chip 42A mounted on the sampling nozzle 42. The processor 16 may be configured to further correct the suction position P1 in a case where the specimen 22 is suctioned by controlling the moving mechanism 46 on the basis of the distal end position b of the chip 42A in the image obtained by capturing the image of the chip 42A mounted on the sampling nozzle 42.

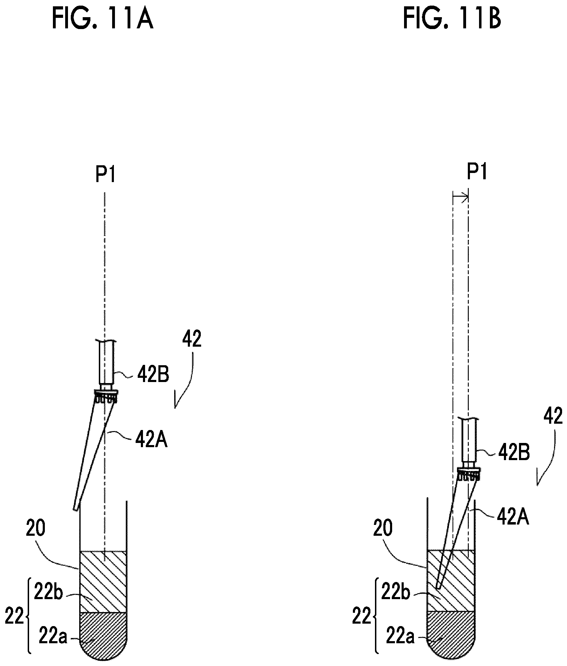

As shown in Fig. 11A, in a case where the chip 42A is mounted while being largely deviated from the nozzle body 42B, the distal end of the chip 42A at the initial suction position P1 may cause the chip 42A to be unable to be inserted into the specimen collection container 20. Therefore, in order to prevent the specimen 22 from being unfavorable, it is preferable to correct the suction position P1 in a case where the specimen 22 is suctioned by controlling the moving mechanism 46 on the basis of the distal end position b of the chip 42A in the image obtained by capturing the image of the chip 42A mounted on the sampling nozzle 42 as shown in Fig. 11B.

In such a manner, the processor 16 may be configured to acquire the image PA of the sampling nozzle 42 on which the chip 42A is mounted from the nozzle imaging camera 61 before the suction of the specimen 22 through the sampling nozzle 42 and correct the suction position P1 in a case where the specimen 22 is suctioned through the sampling nozzle 42. In such a case, it is possible to reliably suction the specimen 22 from the specimen collection container 20.

Further, the processor 16 may be configured to correct the amount of movement in the vertical direction at the suction position P1. That is, a depth position of the distal end of the chip 42A inserted into the specimen collection container 20 in the specimen collection container 20 may be corrected on the basis of the distal end position of the chip 42A in the image PA. In a case where the specimen 22 is blood and is subjected to the centrifugal separation process, the components that can be suctioned are different in accordance with the depth position in the specimen collection container 20. Therefore, in some cases, there is a problem in that, due to the deviation of the chip 42A, a desired component may not be suctioned or an unnecessary component may be mixed in due to a difference in the depth position. Therefore, in a case where the depth position is corrected, it is possible to appropriately suction the desired component, and it is possible to improve the examination accuracy.

### "Examination Apparatus according to Second Embodiment"

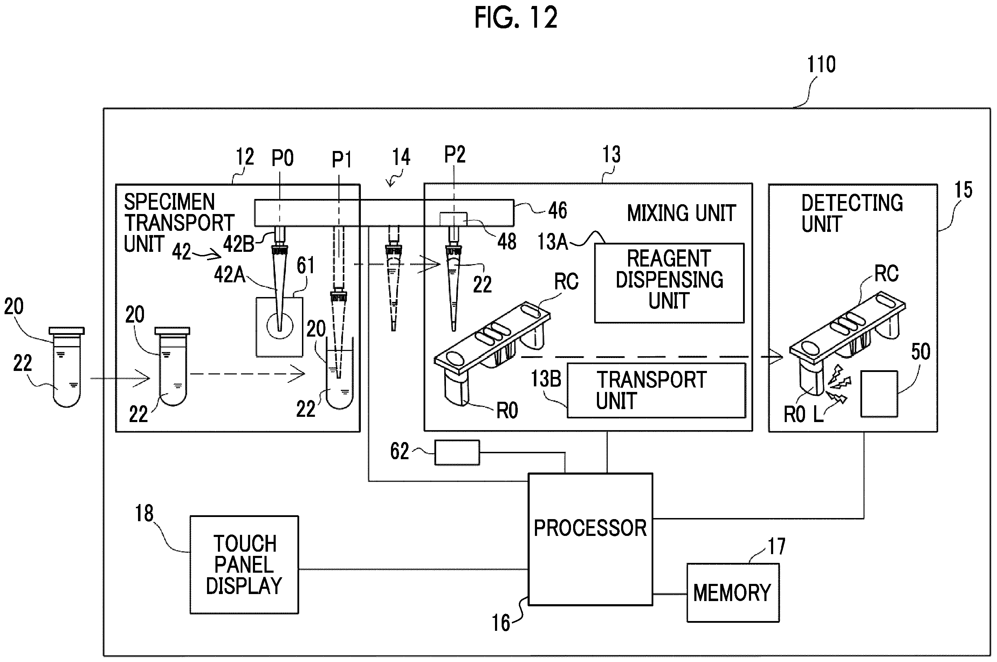

Fig. 12 is a diagram showing a schematic configuration of an examination apparatus 110 according to a second embodiment. The same components in the examination apparatus 10 according to the first embodiment are represented by the same reference numerals, and the detailed description thereof will not be given. Mostly, the differences from the examination apparatus 10 according to the first embodiment will be described.

The examination apparatus 110 according to the second embodiment further includes a reaction cell imaging camera 62 that captures an image of the reaction cell R0. Further, the reaction cell imaging camera 62 is a camera that is provided independently of the nozzle imaging camera 61. The reaction cell imaging camera 62 captures an image of the reaction cell R0 before the specimen 22 is discharged to the reaction cell R0.

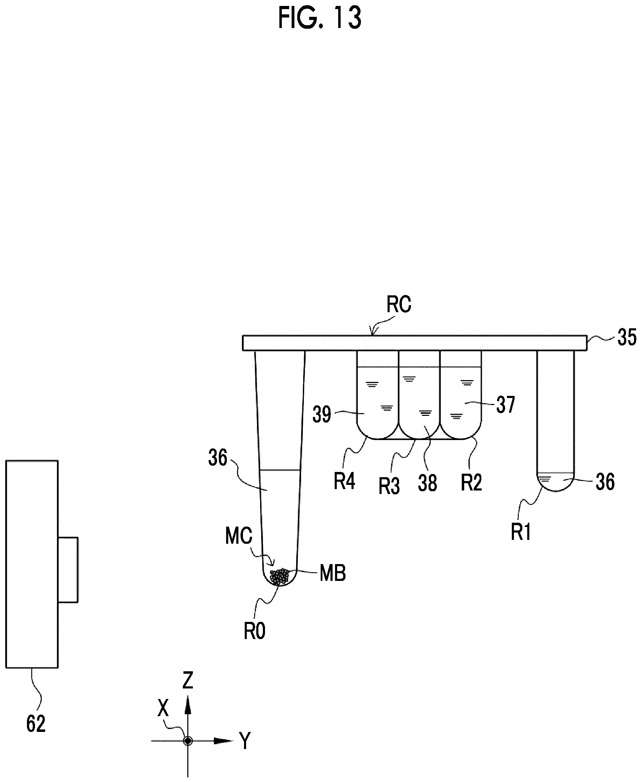

As shown in Fig. 13, the reaction cell imaging camera 62 is disposed to be able to capture an image of the reaction cell R0 disposed at the dispensing position P2. In particular, the lump MC, in which the plurality of magnetic particles MB accommodated in the reaction cell R0 are aggregated, is disposed such that an image of the lump MC can be captured. In the present embodiment, the reaction cell imaging camera 62 captures the image of the reaction cell R0 positioned at the dispensing position P2, where the reaction cell R0 is positioned on the X-Z plane perpendicular to the Y direction.

The processor 16 acquires an image of the reaction cell R0 from the reaction cell imaging camera 62 before the discharging of the specimen 22 to the reaction cell R0 through the sampling nozzle 42, and derives a position of the lump MC of the magnetic particles MB in the reaction cell R0 from the image. Then, the processor 16 corrects the target discharge position α to a position β facing the lump MC in a case where the lump MC deviates from the center of the bottom portion of the reaction cell R0.

In the second embodiment, the processor 16 controls the reaction cell imaging camera 62 such that the reaction cell imaging camera 62 captures an image of the reaction cell R0 after the buffer solution 36 is dispensed to the reaction cell R0 and before the sampling nozzle 42 is inserted into the reaction cell R0.

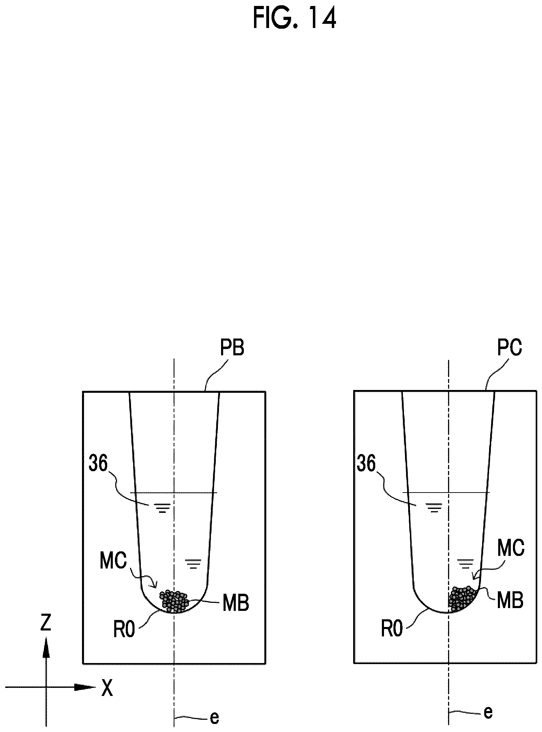

Fig. 14 schematically shows images PB and PC of the reaction cell R0 captured by the reaction cell imaging camera 62. In general, the lump MC of the magnetic particles MB in the reaction cell R0 is positioned at the center of the bottom portion of the reaction cell R0 as shown in the image PB. However, as shown in the image PC, the lump MC may deviate from the center of the bottom portion of the reaction cell R0 during transport of the cartridge RC or the like.

The processor 16 acquires the image PB or PC of the reaction cell R0 from the reaction cell imaging camera 62 and derives the position of the lump MC of the magnetic particles MB in the reaction cell R0. The lump MC is detected from the image PB or PC. Then, the processor 16 corrects the target discharge position α to a position β facing the lump MC in a case where the lump MC deviates from the center of the bottom portion of the reaction cell R0. For example, the centroid position of the region of the lump MC captured in the image is specified, and the deviation of the centroid position from the central axis e of the reaction cell R0 is derived. In a case where the centroid position of the lump MC deviates from the central axis e by a predetermined value or more, it is considered that the lump MC deviates from the center of the bottom portion of the reaction cell R0.

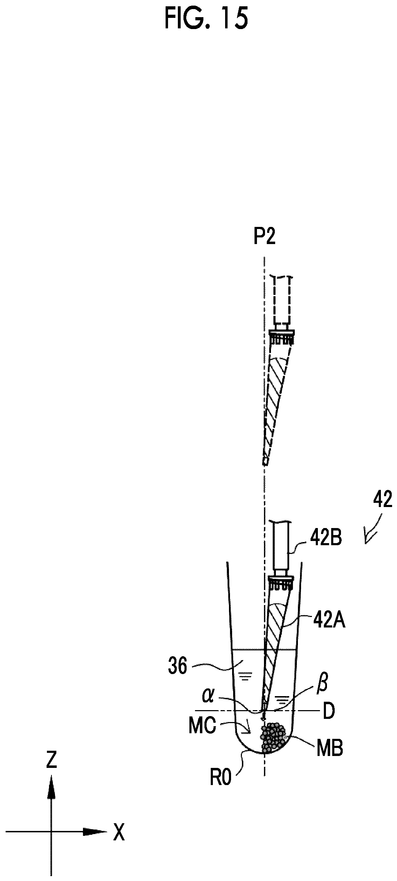

As shown on the image PC, in a case where the lump MC deviates from the center of the bottom portion of the reaction cell R0, the sampling nozzle 42 is moved such that the distal end of the chip 42A is positioned at the target discharge position α as described in the above-mentioned first embodiment. In such a case, a direction, in which the specimen 22 is discharged from the distal end of the chip 42A, may deviate from the lump MC as shown in Fig. 15. In such a case, even in a case where the specimen 22 is discharged from the chip 42A, the water pressure accompanying the discharging of the specimen 22 is not sufficiently applied to the entire lump MC. Thus, a part or the entire lump MC is not dissolved and remains, which may cause failure in mixing.

In the examination apparatus 110 according to the second embodiment, the processor 16 corrects the target discharge position α to the corrected target discharge position β facing the lump MC. Then, the processor 16 controls the moving mechanism 46 such that the moving mechanism 46 moves the sampling nozzle 42 to position the distal end of the chip 42A at the corrected target discharge position β.

Therefore, even in a case where the lump MC of the magnetic particles MB deviates from the center of the bottom portion in the reaction cell R0, the specimen 22 can be discharged toward the lump MC. Therefore, even in a case where the lump MC of the magnetic particles MB deviates from the center of the bottom portion of the reaction cell R0, the lump MC can be dissolved. Thus, it is possible to easily disperse the magnetic particles MB. As a result, it is possible to suppress the failure in mixing and improve the reliability of the examination.

### (Design Modification Example)

As described above, the processor 16 corrects the target discharge position α to the corrected target discharge position β and positions the distal end of the chip 42A at the corrected target discharge position β. Therefore, even in a case where the lump MC of the magnetic particles MB deviates from the center of the bottom portion of the reaction cell R0, it is possible to discharge the specimen 22 toward the lump MC of the magnetic particles MB. However, depending on the inclination of the nozzle body 42B of the chip 42A in the length direction (vertical direction), it is difficult to position the distal end of the chip 42A at the corrected target discharge position β by moving the sampling nozzle 42 in a straight line until the nozzle body 42B abuts on the side wall of the reaction cell R0.

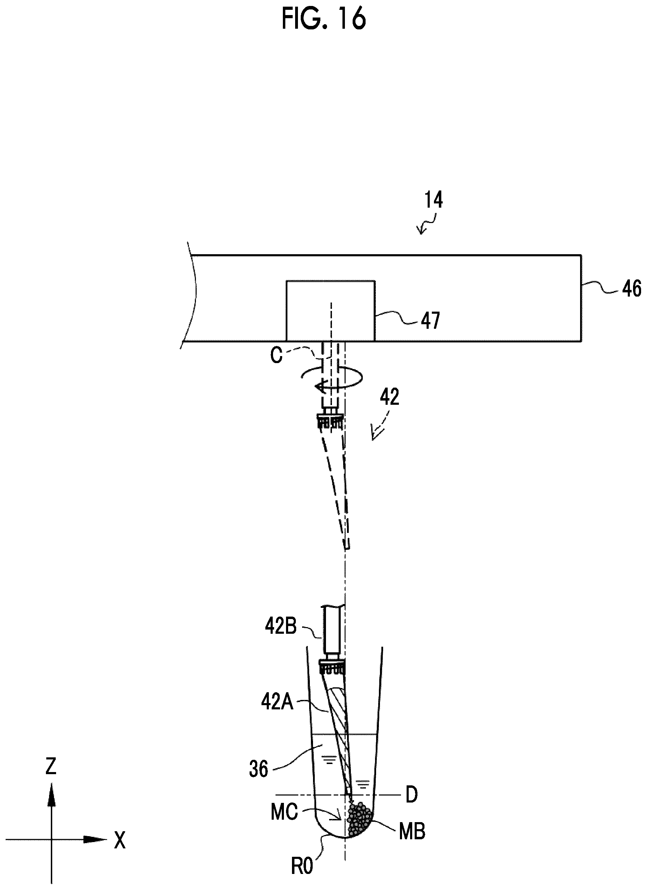

Therefore, as a design modification example, as shown in Fig. 16, the specimen dispensing unit 14 may include a rotation mechanism 47 that rotates the sampling nozzle 42 about the length direction as the axis c. The rotation mechanism 47 can be composed of a rotary motor or the like provided at the base end of the sampling nozzle 42. In a case where the specimen dispensing unit 14 further includes the rotation mechanism 47, the processor 16 corrects the amount of movement such that the distal end position of the chip 42A is set to the position facing the lump MC of the magnetic particles MB by combining the rotation of the sampling nozzle 42 performed by the rotation mechanism 47 and the movement of the sampling nozzle 42 performed by the moving mechanism 46 in the horizontal direction.

The rotation mechanism 47 is particularly effective in the following cases: as shown in Fig. 15, the direction, in which the specimen 22 is discharged, deviates from the vertically downward direction to the inclination direction of the chip 42A due to the inclination of the chip 42A, and the deviation direction of the lump MC of the magnetic particles MB from the center of the bottom portion is different from the direction in which the specimen is discharged from the chip 42A. As shown in Fig. 16, by rotating the sampling nozzle 42, the chip 42A can be positioned such that the distal end of the chip 42A is positioned at a position facing the lump MC of the magnetic particles MB and the discharging direction of the specimen 22 to be discharged is directed to the lump MC of the magnetic particles MB. Thereby, even in a case where the lump MC of the magnetic particles MB deviates from the center of the bottom portion in the reaction cell R0, the lump MC can be dissolved. Thus, it is possible to easily disperse the magnetic particles MB. As a result, it is possible to suppress the failure in mixing and improve the reliability of the examination.

### "Examination Apparatus according to Third Embodiment"

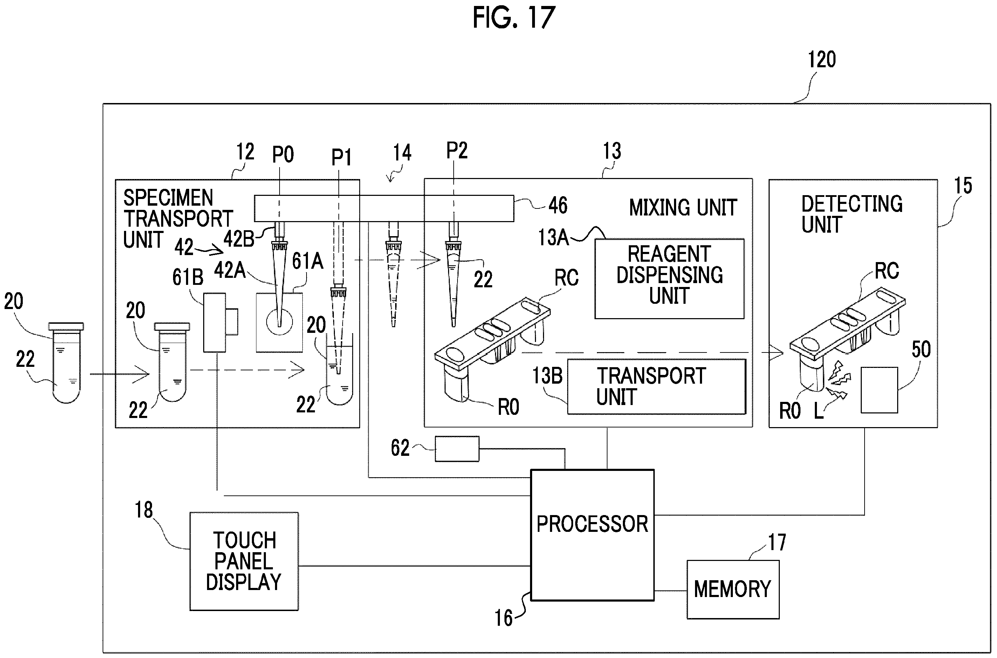

Fig. 17 is a diagram showing a schematic configuration of an examination apparatus 120 according to a third embodiment. The same components in the examination apparatus 110 according to the second embodiment are represented by the same reference numerals, and the detailed description thereof will not be given. Mostly, the differences from the examination apparatus 110 will be described.

The examination apparatus 120 according to the third embodiment includes at least two nozzle imaging cameras including the first camera 61A and the second camera 61B. The first camera 61A and the second camera 61B are disposed around the axis c at intervals of 90° in a case where the length direction of the sampling nozzle 42 is set as the axis c.

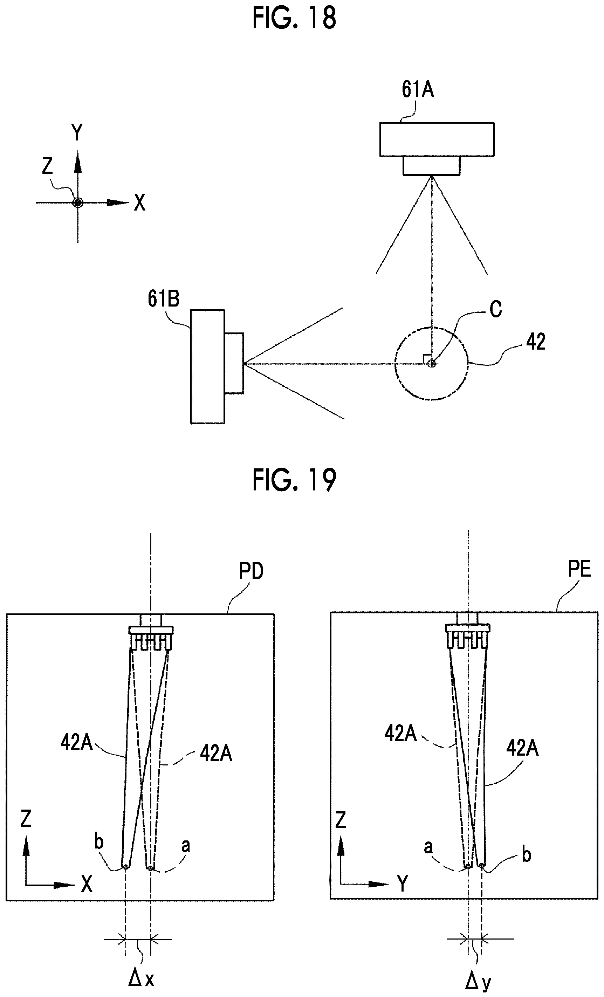

Fig. 18 is a diagram showing a positional relationship of the first camera 61A, the second camera 61B, and the sampling nozzle 42, and is a plan view in a case where a horizontal X-Y plane is observed from the vertical direction. The first camera 61A and the second camera 61B are disposed at positions where images of the chip 42A mounted on the distal end of the nozzle body 42B of the sampling nozzle 42 can be captured. The second camera 61B is disposed at a position which is obtained by rotating the first camera 61A by 90° about the axis c. Similarly to the nozzle imaging camera 61 in the first and second embodiments, the first camera 61A captures the image of the chip 42A on the X-Z plane perpendicular to the Y direction at the mounting position P0 at which the chip 42A is mounted on the nozzle body 42B. The second camera 61B captures an image of the chip 42A on a Y-Z plane perpendicular to the X direction at the mounting position P0.

Fig. 19 is a diagram schematically showing the image PD of the chip 42A captured by the first camera 61A and the image PE of the chip 42A captured by the second camera 61B. As shown in Fig. 19, by providing the first camera 61A and the second camera 61B, not only the amounts of deviations Δx and Δz (refer to Fig. 7) from the appropriate position a in the X direction and the Z direction of the distal end position b of the chip 42A but also the amount of deviation Δy can be accurately detected.

In the examination apparatus 120 of the third embodiment, the processor 16 is configured to control the moving mechanism 46 such that the distal end of the chip 42A is moved to the predetermined target discharge position α inside the reaction cell R0, on the basis of the distal end position of the chip 42A derived from the images PD and PE captured in the two directions.

In such a manner, by providing two nozzle imaging cameras 61A and 61B and capturing the image of the sampling nozzle 42 in a direction orthogonal to the sampling nozzle 42, three-dimensional accurate position information about the distal end position of the chip 42A can be obtained. In such a case, the accuracy of the position correction in a case of moving the sampling nozzle 42 to position the distal end of the chip 42A at the target discharge position α is improved. Thereby, the specimen 22 can be discharged at the target discharge position α of the reaction cell R0 with higher accuracy regardless of the mounting state of the chip 42A. In a case where a lump MC of magnetic particles MB aggregated at the center of the bottom portion of the reaction cell R0 is present, the specimen 22 can be discharged toward the lump MC, and the lump MC can be dissolved. Thus, it is possible to easily disperse the magnetic particles MB. It is possible to suppress the failure in mixing and improve the reliability of the examination.

In a case where only one nozzle imaging camera 61 is provided as in the case of the first embodiment or the second embodiment, the processor 16 may derive the distal end position of the chip 42A in the depth direction from the nozzle imaging camera 61 toward the chip 42A on the basis of the degree of the optical blurring of the chip 42A appearing in the image in addition to a position within the image at the distal end position of the chip 42A, and may correct the target discharge position α.

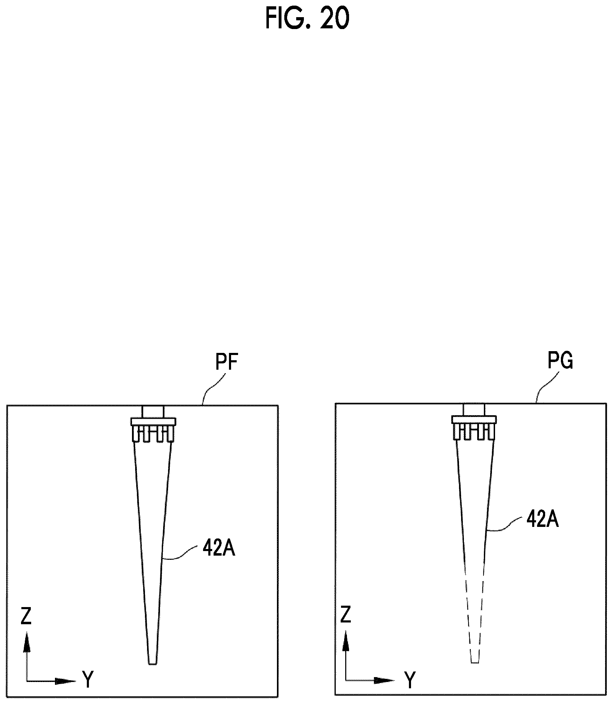

The nozzle imaging camera 61 is set to perform focusing on the chip 42A in a state where the chip 42A is appropriately mounted. Fig. 20 is a diagram schematically showing an image PF and an image PG of the chip 42A captured by the nozzle imaging camera 61. In a case where the chip 42A is appropriately mounted on the nozzle body 42B, as shown in the image PF in Fig. 20, an image of the entire chip 42A is captured without blurring. In contrast, in a case where the chip 42A is mounted to be inclined in the depth direction (here, the X direction) from the nozzle imaging camera 61 toward the chip 42A, a part of the image PG in Fig. 20 indicated by a broken line deviates from the focus and is captured in a blurred state. Therefore, it is possible to derive the amount of deviation of the distal end position of the chip 42A from the appropriate position in the X direction on the basis of the degree of the optical blurring. A degree of optical blurring can be derived by acquiring spatial frequency components of an image and performing an analysis process or the like.

The processor 16 measures the degree of optical blurring of the chip 42A in the image PG to derive the distal end position of the chip 42A in the depth direction from the nozzle imaging camera 61 toward the chip 42A. Thereby, it is possible to obtain the three-dimensional accurate position information about the distal end position of the chip 42A. Accordingly, it is possible to obtain the same effect as in a case where two or more nozzle imaging cameras are provided. On the other hand, it is possible to minimize the equipment costs as compared with a case where two cameras are provided.

In a case where only one nozzle imaging camera 61 is provided, the method of deriving the distal end position of the chip 42A in the depth direction toward the chip 42A from the nozzle imaging camera 61 is not limited to the method of deriving the distal end position on the basis of the degree of the optical blurring of the chip 42A appearing in the image.

The nozzle imaging camera 61 is set such that the distal end of the chip 42A mounted appropriately is brought into focus. In a case where the chip 42A is mounted in a deviated manner and the distal end of the chip 42A deviates from the appropriate position, the distal end of the chip 42A deviates from the focal position. Therefore, the processor 16 may be configured to control the nozzle imaging camera 61 to change the focal position to the distal end position of the chip 42A and derive the amount of deviation of the distal end position b of the chip 42A from the appropriate position a from the amount of change of the focal position.

In such a case, it is also possible to obtain the three-dimensional accurate position information about the distal end position of the chip 42A. Accordingly, it is possible to obtain the same effect as in a case where two or more nozzle imaging cameras are provided. On the other hand, it is possible to minimize the equipment costs as compared with a case where two cameras are provided.

In each of the embodiments, the examination apparatus that detects the target substance by detecting luminescence or fluorescence from the label attached to the target substance by using the antigen-antibody reaction has been described. However, the invention of the present disclosure can also be applied to an examination apparatus that performs the biochemical analysis by using a reaction other than the antigen-antibody reaction. Further, the present disclosure can be applied to an examination apparatus having a configuration in which the distal end of the chip is positioned at the predetermined target discharge position α inside the reaction cell R0.

In the cartridge RC used in the examination apparatuses 10, 110, and 120 according to the above-mentioned embodiments, the enzyme is used as the label S, and the detecting unit 15 is configured to detect the luminescence generated by the reaction between the enzyme and the luminescent reagent. However, the label S is not limited to the enzyme and may be a fluorescent label such as a fluorescent dye or fluorescent beads. In a case where the flag S is a fluorescent flag, the detecting unit 15 may be provided with an excitation light source that irradiates the fluorescent flag with excitation light and a photodetector that detects fluorescence generated from the fluorescent flag.

In each of the above-mentioned embodiments, as hardware structures of processing units that execute various types of process as the internal configuration of the processor 16, the following various processors can be used. The various processors include, for example, a CPU which is a general-purpose processor executing software to function as various processing units as described above, a programmable logic device (PLD), such as a field programmable gate array (FPGA), which is a processor whose circuit configuration can be changed after manufacture, or a dedicated electric circuit, such as an application specific integrated circuit (ASIC), which is a processor having a dedicated circuit configuration designed to perform a specific process.

One processing unit may be constituted by one of the various processors, or may be a combination of two or more processors of the same type or different types (for example, a combination of a plurality of FPGAs and/or a combination of a CPU and an FPGA). The plurality of processing units may be configured of one processor.

As an example of the plurality of processing units composed of one processor, first, as represented by computers such as a client and a server, there is a form in which one processor is composed of a combination of one or more CPUs and software and this processor functions as a plurality of processing units. A second example of the configuration is an aspect in which a processor that implements the functions of the whole system including a plurality of processing units using one integrated circuit (IC) chip is used. A representative example of this aspect is a system-on-chip (SoC). As described above, the various processing units are configured using one or more of the various processors as the hardware structure.

More specifically, a circuitry combining circuit elements such as semiconductor elements may be used as the hardware structure of the various processors.

The present disclosure is not limited to the above-mentioned embodiments, and can be implemented with appropriate modifications, such as omitting a configuration or replacing a configuration with a different configuration within the scope that does not deviate from the gist of the present disclosure.

The disclosure of JP2022-043032 filed on March 17, 2022 is incorporated herein by reference in its entirety.

All documents, patent applications, and technical standards described in the present specification are incorporated into the present specification by reference to the same extent as in a case where the individual documents, patent applications, and technical standards were specifically and individually stated to be incorporated by reference.

## Claims

1. An examination apparatus comprising:
a detecting unit that detects a target substance in a specimen;
a specimen dispensing unit that has a sampling nozzle, through which the specimen is suctioned from a specimen collection container and the specimen is discharged to a reaction cell and on which a chip is mounted at a distal end thereof, and a moving mechanism which moves the sampling nozzle;
a nozzle imaging camera that captures an image of the sampling nozzle on which the chip is mounted; and
a processor that is configured to control the moving mechanism and the nozzle imaging camera,
wherein the processor is configured to acquire the image of the sampling nozzle on which the chip is mounted from the nozzle imaging camera before the specimen is discharged to the reaction cell through the sampling nozzle, and
the processor is configured to control the moving mechanism on the basis of a distal end position of the chip in the image such that the moving mechanism moves a distal end of the chip to a predetermined target discharge position inside the reaction cell.

2. The examination apparatus according to claim 1,
wherein the target discharge position is a position which is at a center of the reaction cell in a horizontal direction and from which a bottom portion of the reaction cell is spaced by an interval predetermined as a reference in a vertical direction.

3. The examination apparatus according to claim 1 or 2,
wherein the processor is configured to:
acquire an image of the distal end of the chip by causing the nozzle imaging camera to capture the image before inserting the sampling nozzle into the reaction cell;
derive, from the image, an amount of deviation between the distal end position of the chip and a predetermined appropriate position as a position at which the chip is appropriately mounted on the sampling nozzle; and
control the moving mechanism such that the moving mechanism corrects the amount of deviation and moves the distal end of the chip to the target discharge position.

4. The examination apparatus according to any one of claims 1 to 3, further comprising:
a notification unit that issues a notification of an error,
wherein an allowable range is set in which an amount of deviation between the distal end position of the chip and a predetermined appropriate position as a position at which the chip is appropriately mounted on the sampling nozzle is allowable, and
the processor is configured to:
derive, from the image, the amount of deviation between the distal end position of the chip and the appropriate position; and
stop movement of the sampling nozzle by the moving mechanism and cause the notification unit to issue the notification of the error, in a case where the amount of deviation is out of the allowable range.

5. The examination apparatus according to any one of claims 1 to 4, further comprising:
a reaction cell imaging camera that captures an image of the reaction cell,
wherein the reaction cell accommodates a lump in which a plurality of magnetic particles are aggregated, and
the processor is configured to:
acquire the image of the reaction cell from the reaction cell imaging camera before the specimen is discharged to the reaction cell through the sampling nozzle;
derive a position of the lump in the reaction cell from the image; and
correct the target discharge position to a position facing the lump in a case where the lump deviates from a center of a bottom portion of the reaction cell.

6. The examination apparatus according to any one of claims 1 to 5,
wherein the specimen dispensing unit includes a rotation mechanism that rotates the sampling nozzle about a length direction as an axis.

7. The examination apparatus according to any one of claims 1 to 6,
wherein at least two cameras including a first camera and a second camera are provided as the nozzle imaging camera, and
the first camera and the second camera are disposed around an axis at an interval of 90° in a case where a length direction of the sampling nozzle is set as the axis.

8. The examination apparatus according to any one of claims 1 to 7,
wherein the processor is configured to derive the distal end position of the chip in a depth direction from the nozzle imaging camera toward the chip and correct the target discharge position on the basis of a degree of optical blurring of the chip appearing in the image in addition to the distal end position of the chip in the image.

9. The examination apparatus according to any one of claims 1 to 8,
wherein the processor is configured to:
acquire the image from the nozzle imaging camera before the specimen is suctioned through the sampling nozzle; and
correct a suction position of the sampling nozzle in a case where the specimen is suctioned through the sampling nozzle by controlling the moving mechanism on the basis of the distal end position of the chip in the image.

10. The examination apparatus according to any one of claims 1 to 9,
wherein the detecting unit detects the target substance by detecting luminescence or fluorescence from a label attached to the target substance by using an antigen-antibody reaction.
